(19) Europäisches Patentamt
European Patent Office
Office européen des brevets

(11) **EP 4 317 973 A1**

(12) **EUROPEAN PATENT APPLICATION**
published in accordance with Art. 153(4) EPC

(43) Date of publication:
**07.02.2024 Bulletin 2024/06**

(51) International Patent Classification (IPC):
***G01N 33/543*** (2006.01)

(21) Application number: **22775170.8**

(52) Cooperative Patent Classification (CPC):
**G01N 21/8483; G01N 33/543;** G01N 21/78

(22) Date of filing: **10.03.2022**

(86) International application number:
**PCT/JP2022/010715**

(87) International publication number:
**WO 2022/202378 (29.09.2022 Gazette 2022/39)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **26.03.2021 JP 2021054135**

(71) Applicant: **FUJIFILM Corporation
Tokyo 106-8620 (JP)**

(72) Inventor: **ISHII, Hiroyasu
Ashigarakami-gun, Kanagawa 258-8538 (JP)**

(74) Representative: **Hughes, Andrea Michelle
Dehns Germany
Theresienstraße 6-8
80333 München (DE)**

(54) **TEST CARTRIDGE**

(57) An assay cartridge is an assay cartridge that is attachably and detachably loaded into an immunochromatographic assay apparatus, including a carrier having an assay region in which a color development state changes depending on whether a sample is positive or negative, a case in which the carrier is accommodated, and an opening portion that is provided in the case, is used for observing an observation region LA including the assay region, and is illuminated by a light source in the immunochromatographic assay apparatus, in which at least a part of an inner peripheral wall 18A of the opening portion is a processed surface having an angle of 50° or more with respect to a surface of the observation region LA and having been subjected to a roughening treatment.

FIG. 7

EP 4 317 973 A1

**Description**

## BACKGROUND OF THE INVENTION

### 1. Field of the Invention

The technique of the present disclosure relates to an assay cartridge.

### 2. Description of the Related Art

The following JP2013-238543A describes an assay device (referred to as a test piece in JP2013-238543A) for performing an assay of whether a sample is positive or negative, that is, whether or not the sample contains a test substance, using an immunochromatographic method. Such an assay device is referred to as an assay cartridge or the like. The assay cartridge includes a carrier having an assay region for assaying whether a sample is positive or negative, and a case for accommodating the carrier. An antibody that reacts with the test substance is immobilized in the assay region, and the color development state of the assay region changes in a case where the antibody reacts with the test substance. In the case, an opening portion for observing the color development state of the assay region from the outside is formed.

## SUMMARY OF THE INVENTION

In the assay cartridge described in JP2013-238543A, the side wall of the opening portion is an inclined surface formed in a tapered shape. In a state where the assay cartridge is loaded in the assay apparatus, a detection unit that detects the reflected light reflected in the assay region is disposed at a directly-facing position directly facing the opening portion. In addition, a light source is disposed on the side of the detection unit, and the light source is located obliquely upward with respect to the opening portion. The light from the light source is emitted from obliquely upward with respect to the opening.

In the assay cartridge described in JP2013-238543A, the angle of the inclined surface of the side wall of the opening portion is about 45° with respect to the surface of the assay region, and the antireflection layer is formed on the inclined surface. First, the side wall is formed as an inclined surface such that light can easily enter the assay region. Then, by forming the antireflection layer on the inclined surface, unnecessary light reflected by the side wall is prevented from entering the detection unit.

In a case where the detection unit is disposed at a directly-facing position directly facing the opening portion as described in JP2013-238543A, the opening portion must be irradiated with light from an oblique direction. In the case of being irradiated with light from an oblique direction in this way, there has been a problem that the light quantity distribution in the observation region tends to be uneven, caused by that a difference in distance from the light source occurs in the observation region including the assay region and the peripheral region thereof, that the light is vignetted in the periphery of the opening portion, or the like. The configuration of the assay cartridge described in JP2013-238543A is not sufficient to solve such a problem.

In consideration of the above-described facts, the technique according to the present disclosure provides an assay cartridge in which the light quantity distribution of the observation region including the assay region and the peripheral region thereof is likely to be even in a case where the opening portion is illuminated, as compared with the prior art.

The assay cartridge of the present disclosure is an assay cartridge that is attachably and detachably loaded into an immunochromatographic assay apparatus, including a carrier having an assay region in which a color development state changes depending on whether a sample is positive or negative, a case in which the carrier is accommodated, and an opening portion that is provided in the case, is used for observing an observation region including the assay region, and is illuminated by a light source in the immunochromatographic assay apparatus, in which at least a part of an inner peripheral wall of the opening portion is a processed surface having an angle of 50° or more with respect to a surface of the observation region and having been subjected to a roughening treatment.

In the assay cartridge of the present disclosure, the angle is preferably in a range of 70° or more and 90° or less.

In the assay cartridge of the present disclosure, it is preferable that the opening portion has a rectangular shape and the processed surface is formed on a wall surface of the inner peripheral wall extending in a longitudinal direction.

In the assay cartridge of the present disclosure, the assay region is a line-shaped assay line, and the assay line is disposed along a direction intersecting the longitudinal direction.

In the assay cartridge of the present disclosure, the processed surface is preferably formed over an entire circumference of the inner peripheral wall.

In the assay cartridge of the present disclosure, among parameters defining surface roughness of the processed surface, an arithmetic average height Sa is preferably 100 $\mu$m or less.

In the assay cartridge of the present disclosure, the roughening treatment is preferably an emboss processing.

In the assay cartridge of the present disclosure, the opening portion has a rectangular shape, and in a case where an opening width in the direction along the surface of the observation region and in a short side direction of the opening portion is denoted as D, and a height of the inner peripheral wall in the direction orthogonal to the opening width is denoted as H, $0.5D \leq H \leq 1.5D$.

In a case where the inner peripheral wall of the opening portion is a first inner peripheral wall, the assay cartridge of the present disclosure may have a second inner peripheral wall that rises in a direction away from the surface along an intersection direction intersecting the surface of the observation region, around the opening portion.

In the assay cartridge of the present disclosure, a plane portion extending in a direction along the surface of the observation region may be provided around the opening portion, and the second inner peripheral wall may rise from an end edge of the plane portion on a side opposite to the opening portion.

In the assay cartridge of the present disclosure, in a case where the inner peripheral wall of the opening portion is the first inner peripheral wall, the second inner peripheral wall may rise from an end edge of the first inner peripheral wall.

In the assay cartridge of the present disclosure, at least a part of the second inner peripheral wall preferably is subjected to the roughening treatment.

In the assay cartridge of the present disclosure, the second inner peripheral wall may be inclined at an angle smaller than the angle of the first inner peripheral wall.

In the assay cartridge of the present disclosure, it is preferable that a structure having a convex shape in a direction away from the surface of the observation region is provided around the opening portion and at least a part of an outer peripheral wall of the structure is subjected to the roughening treatment.

According to the assay cartridge of the present disclosure, the light quantity distribution of the observation region including the assay region and the peripheral region thereof is likely to be even in a case where the opening portion is illuminated, as compared with the prior art.

**BRIEF DESCRIPTION OF THE DRAWINGS**

Fig. 1 is a perspective view of an assay cartridge according to the present disclosure.
Fig. 2 is an exploded perspective view of the assay cartridge according to the present disclosure.
Fig. 3A is a cross-sectional view showing a state in which a first pressing operation part of the assay cartridge according to the present disclosure is operated, and Fig. 3B is a cross-sectional view showing a state in which the first pressing operation part and a second pressing operation part are operated.
Fig. 4 is a side view showing a positional relationship between an assay strip, a multifunctional member, a first reagent holding part, and a second reagent holding part, in the assay cartridge according to the present disclosure.
Fig. 5 is an explanatory diagram of an immunochromatographic method.
Fig. 6 is a perspective view showing an appearance of an immunochromatographic assay apparatus into which the assay cartridge according to the present disclosure is loaded.
Fig. 7 is a partially broken side view showing a positional relationship between a light source and an observation window in an assay apparatus in a state where an assay cartridge according to the present disclosure is loaded.
Fig. 8 is a perspective view showing the observation window of the assay cartridge according to the present disclosure.
Fig. 9 is a top view showing the observation window of the assay cartridge according to the present disclosure.
Fig. 10 is a cross-sectional view showing an observation window of the assay cartridge according to the present disclosure.
Fig. 11 is a cross-sectional view showing an example of light incident from a light source into the observation region of the assay cartridge according to the present disclosure.
Fig. 12 is a cross-sectional view showing another example of light incident from a light source into the observation region of the assay cartridge according to the present disclosure.
Fig. 13 is a cross-sectional view showing an example of incident light to an inner peripheral wall and reflected light from the inner peripheral wall of the observation window of the assay cartridge according to the present disclosure.
Fig. 14 is a cross-sectional view showing a comparative example.
Fig. 15 is a cross-sectional view showing an example of a relationship between a width and a height of an observation window of an assay cartridge according to the present disclosure.
Fig. 16 is a cross-sectional view showing a modified example of an angle of an inner peripheral wall of an observation window of an assay cartridge according to the present disclosure.
Fig. 17 is a cross-sectional view showing a state in which an incident angle of light is changed in a modified example shown in Fig. 16.
Fig. 18 is a cross-sectional view showing a modified example in which the first inner peripheral wall and the second inner peripheral wall are formed as the inner peripheral wall of the observation window of the assay cartridge according to the present disclosure.
Fig. 19 is a cross-sectional view showing an example of incident light and reflected light in a modified example in which the first inner peripheral wall and the second inner peripheral wall are formed as the inner peripheral wall.

Fig. 20 is a cross-sectional view showing a modified example in which an angle of the second inner peripheral wall is changed.

Fig. 21 is a cross-sectional view showing a modified example in which angles of the first inner peripheral wall and the second inner peripheral wall are changed.

Fig. 22 is a cross-sectional view showing a modified example in which a flat plane portion is provided between the first inner peripheral wall and the second inner peripheral wall.

Fig. 23 is a cross-sectional view showing a modified example in which the observation region of the assay cartridge according to the present disclosure receives transmitted light.

## DESCRIPTION OF THE PREFERRED EMBODIMENTS

Hereinafter, an assay cartridge according to an embodiment of the present invention will be described with reference to the drawings. The constituent elements indicated by the same reference numerals in the drawings mean the same constituent elements. However, unless otherwise specified in the specification, each component is not limited to one, and a plurality of each component may be present.

In addition, description of overlapping configurations and reference numerals in the respective drawings may be omitted. The present invention is not limited to the following embodiments, and can be implemented with appropriate modifications, such as omitting a configuration or replacing a configuration with a different configuration within the scope of the object of the present invention.

The directions indicated by the arrows X and Y, which are appropriately shown in the respective figures, are directions along the horizontal plane and are orthogonal to each other. In addition, the direction indicated by the arrow Z is a direction along the perpendicular direction (vertical direction). The directions indicated by the arrows X, Y, and Z in respective figures coincide with each other.

<Overview of assay cartridge>

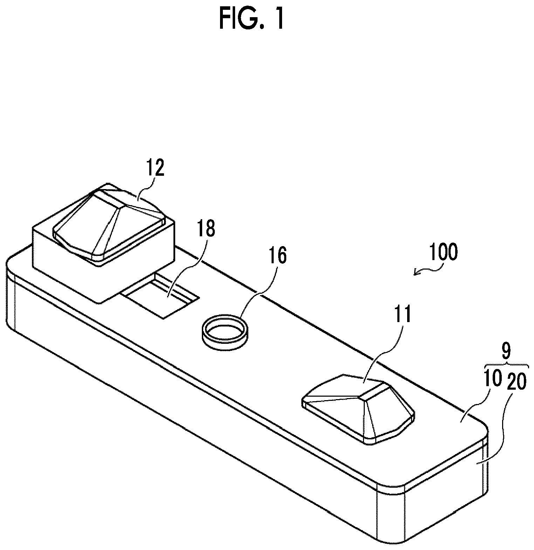

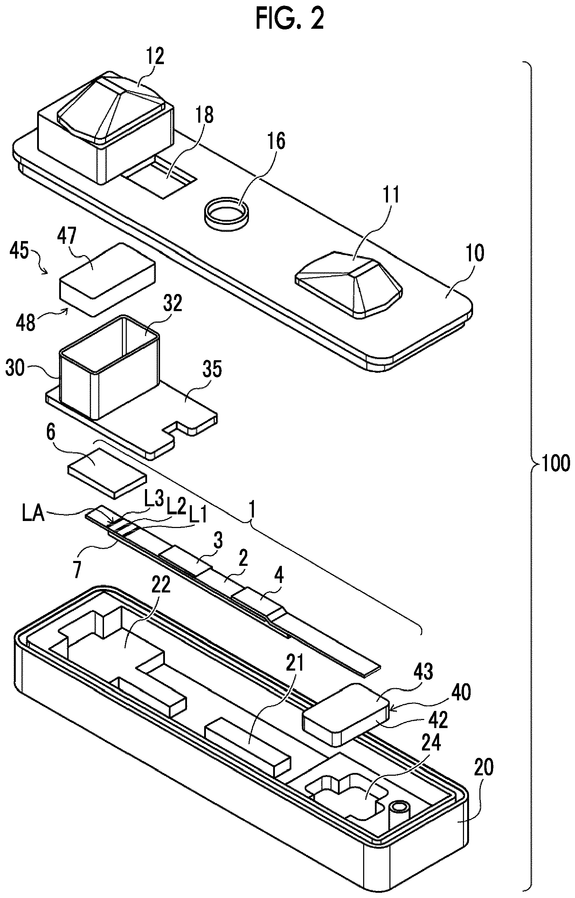

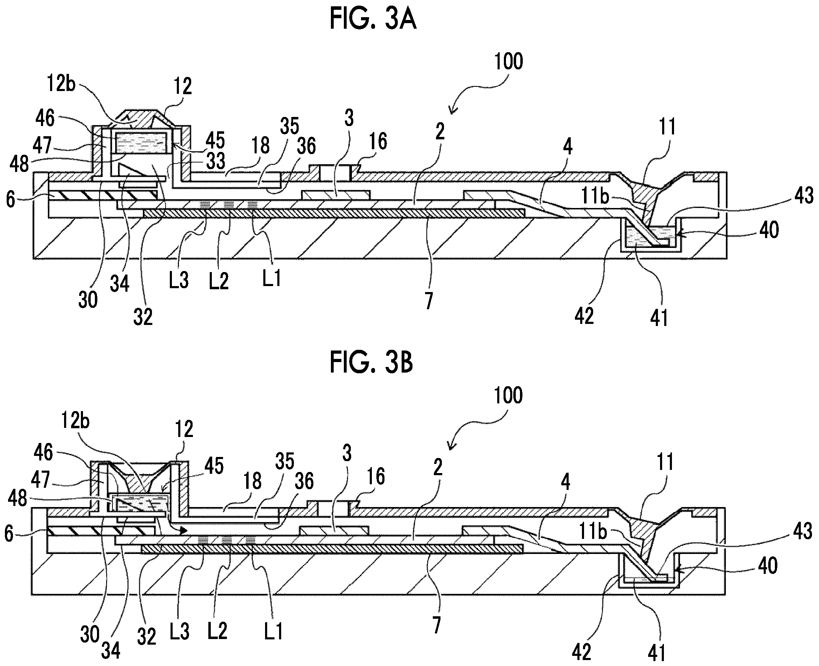

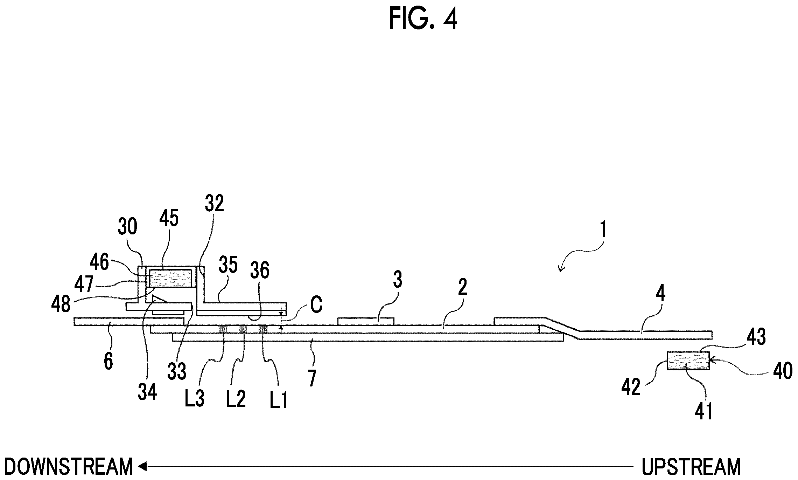

Fig. 1 is an external view of a cartridge 100 which is an assay cartridge according to one embodiment, and Fig. 2 is an exploded perspective view of the cartridge 100. Fig. 3 is a diagram showing a state in which the first pressing operation part 11 and the second pressing operation part 12 provided in the cartridge 100 are operated. Fig. 4 is a diagram showing the main accommodated components in the cartridge 100.

The cartridge 100 is a single-use type that is used one by one in each sample of assay target. As shown in Fig. 2, an assay strip 1 including an immunochromatographic carrier 2 (hereinafter, referred to as a carrier 2) is provided in the cartridge 100. An assay region L1 is provided in the carrier 2, and the color development state changes depending on whether or not the sample contains a test substance, that is, whether the sample is positive or negative.

The "change in color development state" includes any of an aspect in which a first color different from the color of the carrier 2 changes to another second color (that is, a color change), an aspect in which the color of the carrier 2 changes to another color by developing a color different from that of the carrier 2 (that is, color development), and an aspect in which the density of the color changes (that is, a change in density).

The sample is simply required to be a specimen that may contain a test substance, and the sample is not particularly limited. The sample is, for example, a biological specimen, particularly body fluid or excrement of an animal (particularly, a human) such as blood, serum, blood plasma, spinal fluid, tear fluid, sweat, urine, pus, nasal mucus, nasal swab, throat swab, nasal aspirate, or sputum, an organ, a tissue, a mucous membrane and skin, or swabs containing them, or a liquid specimen containing animals and plants themselves or a dried body thereof. Examples of the test substance include an antigen, an antibody, a protein, and a low-molecular-weight compound.

The cartridge 100 has a configuration that allows a user to visually confirm whether the sample is positive or negative. Such a cartridge 100 is also referred to as an immunochromatographic assay tool, an immunochromatographic assay kit, or the like.

As shown in Fig. 1 and Fig. 2, as an example, the cartridge 100 includes a case 9 constituted of a case body 20 and a cover member 10. The case 9 is formed of, for example, a resin material. An opening is formed in an upper part of the case body 20, and in addition to the assay strip 1, a first reagent holding part 40, a second reagent holding part 45, and the like are accommodated therein. The cover member 10 covers the opening of the case body 20 by being attached to the opening part of the case body 20. The case 9 has an elongated shape as a whole in accordance with the elongated shape of the assay strip 1.

In the present example, a dropping port 16, an observation window 18, a first pressing operation part 11, and a second pressing operation part 12 are provided on an upper part of the case 9 constituted of the cover member 10. Each of these parts is integrally molded with the cover member 10 as an example. The dropping port 16 is an opening for adding dropwise a sample into the inside of the case 9. A boss is vertically provided on the edge of the dropping port 16 toward the upper part.

(Observation window)

An observation window 18 is an opening portion for observing the assay region L1 from the outside, in the present example, the size of the observation window 18 is a size such that, in addition to the assay region L1, the control region L2 and the color development region L3, which will be described later, can also be observed. In the carrier 2, a region that includes the assay region L1, the control region L2, the color development region L3, and the peripheral region thereof and can be observed from the observation window 18 is referred to as an observation region LA. The user can confirm the observation region LA through the observation window 18. The user can confirm whether the sample is positive or negative by observing the color development state of the assay region L1 in the observation region LA.

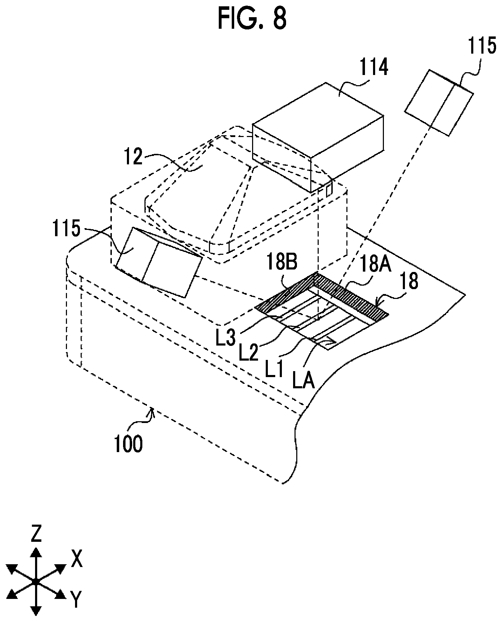

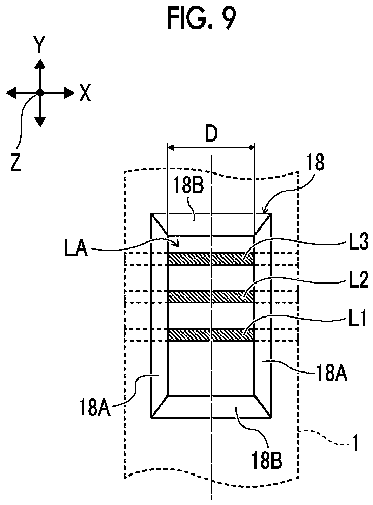

As shown in Fig. 8 and Fig. 9 which will be described later, the cartridge 100 of the present example is characterized in the observation window 18, but the characteristic of the observation window 18 will be described after a basic configuration, a basic method for using, and the like of the cartridge 100 is described.

(First pressing operation part, second pressing operation part)

As shown in Fig. 2 and Fig. 3, the first pressing operation part 11 is an operating part operated to supply the first reagent 41 in the first reagent holding part 40 to the carrier 2. The second pressing operation part 12 is an operating part operated to supply the second reagent 46 in the second reagent holding part 45 to the carrier 2. As will be described later, the first reagent 41 and the second reagent 46 are amplifying liquids for amplifying the color development in the assay region L1 in a case where the sample is positive.

As shown in Fig. 3A, in a case where a pressing force is applied from the outside as an external force to the first pressing operation part 11 by a pressing operation by a user or the like, the first pressing operation part 11 is deformed. As shown in Fig. 2, as an example, the first pressing operation part 11 has a quadrangular pyramid shape, and in a case where a pressing force is applied from above to a region including the apex of the quadrangular pyramid, as shown in Fig. 3A, the first pressing operation part 11 is deformed such that the apex of the quadrangular pyramid sinks into the inside of the case 9. In a case of where the first pressing operation part 11 is deformed in this manner, a pressing force is applied to the first reagent holding part 40 inside the case 9. In the first reagent holding part 40, deformation or the like due to a pressing force applied through the first pressing operation part 11 occurs. Due to this deformation or the like, the first reagent 41 held by the first reagent holding part 40 is supplied to the assay strip 1.

In addition, the first pressing operation part 11 is deformed by pressing and then the deformed state is maintained. Accordingly, after the first pressing operation part 11 is pressed, the supply of the first reagent 41 to the assay strip 1 is continued.

Similarly, as shown in Fig. 3B, in a case where a pressing force is applied from the outside as an external force to the second pressing operation part 12, the second pressing operation part 12 is deformed. As shown in Fig. 2, similarly to the first pressing operation part 11, the second pressing operation part 12 of the present example also has a quadrangular pyramid shape, and in a case where a pressing force is applied from above to a region including the apex of the quadrangular pyramid, as shown in Fig. 3B, the second pressing operation part 12 is deformed such that the apex of the quadrangular pyramid sinks into the inside of the case 9. In a case of where the second pressing operation part 12 is deformed in this manner, a pressing force is applied to the second reagent holding part 45 inside the case 9. In the second reagent holding part 45, deformation or the like due to a pressing force applied through the second pressing operation part 12 occurs. Due to this deformation or the like, the second reagent 46 held by the second reagent holding part 45 is supplied to the assay strip 1. In the second pressing operation part 12 of the present example, an abutting part 12b that abuts on the second reagent holding part 40 is provided in the inside of the case 9.

(First reagent holding part)

As shown in Fig. 2 and Fig. 3, the case body 20 accommodates the assay strip 1 including the carrier 2 along the longitudinal direction. As shown in Fig. 3 and Fig. 4, in the case body 20, the first reagent holding part 40 is disposed on one end part side (upstream side shown in Fig. 4) in the longitudinal direction. In the case body 20, in a portion where the first reagent holding part 40 is disposed, the first accommodating part 24 that is a recess-shaped in accordance with the shape of the first reagent holding part 40 is formed. One end part of the assay strip 1 is disposed above the first reagent holding part 40 in a state of being accommodated in the first accommodating part 24.

As shown in Fig. 3 and Fig. 4, the first reagent holding part 40 holds the first reagent 41. The first reagent holding part 40 is constituted of, for example, a container 42 formed of a resin material and having an opening on one surface, and a sheet member 43 that covers the opening of the container 42 and is breakable. The container 42 is filled with the first reagent 41, and the opening of the container 42 is sealed by the sheet member 43. The first reagent holding part 40 is disposed in the first accommodating part 24 in a posture in which the sheet member 43 faces upward.

The pressing force applied from the first pressing operation part 11 is transmitted to the sheet member 43 of the first reagent holding part 40 via the end part of the assay strip 1 to break the sheet member 43. The sheet member 43 is broken, and thus the first reagent 41 is supplied to the assay strip 1. In the first pressing operation part 11 of the present example, a protruding part 11b that abuts on the sheet member 43. The protruding part 11b has, for example, an elongated shape extending in the longitudinal direction in the width direction of the assay strip 1 and a pointed shape toward the sheet member 43, such that the sheet member 43 is easily broken.

(Multifunctional member)

In addition, the cartridge 100 includes a multifunctional member 30 having a function of accommodating the second reagent holding part 45. The multifunctional member 30 is disposed on the other end part side (downstream side shown in Fig. 4) of the case body 20 and above the assay strip 1. The multifunctional member 30 is a member in which the second accommodating part 32 and the flow channel forming part 35 are integrally formed. The second accommodating part 32 is a part accommodating the second reagent holding part 45. The second accommodating part 32 has a box shape having an opened upper surface. As shown in Fig. 4, on the bottom of the second accommodating part 32, a protrusion 34 for breaking a sheet member 48, which will be described later, of the second reagent holding part 45, and an opening 33 that allows to flow the second reagent 46 flowed out from the second reagent holding part 45, toward the assay strip 1.

Furthermore, the flow channel forming part 35 is provided to be connected to the upstream side from the second accommodating part 32. The flow channel forming part 35 has a flat plate shape, is disposed at a position facing the assay region L1 or the like in the longitudinal direction of the assay strip 1, and is disposed with an interval from the assay strip 1. Then, between the flow channel forming part 35 and the assay strip 1, a flow channel for flowing the second reagent 46 flowed out from the second accommodating part 32 toward the assay region L1 or the like is formed. In addition, the flow channel forming part 35 is disposed between the observation window 18 and the assay region L1 or the like of the assay strip 1. Therefore, the flow channel forming part 35 is formed of a transparent member and thus the assay region L1 and the like can be observed through the observation window 18.

(Second reagent holding part)

The second reagent holding part 45 holds the second reagent 46. The second reagent holding part 45 is constituted of, for example, a container 47 formed of a resin material and having an opening on one surface, and a sheet member 48 that covers the opening of the container 47 and is breakable. The container 47 is filled with the second reagent 46, and the opening of the container 47 is sealed by the sheet member 48. The second reagent holding part 45 is disposed in the second accommodating part 32 in a posture in which the sheet member 48 faces downward. Accordingly, the sheet member 48 faces the protrusion 34 in the second accommodating part 32.

The pressing force applied from the second pressing operation part 12 to the second reagent holding part 45 acts in a direction of pushing down the second reagent holding part 45 downwardly, whereby the sheet member 48 is pressed against the protrusion 34. The sheet member 48 is pressed against the protrusion 34 to break the sheet member 48. The sheet member 48 is broken, and thus the second reagent 46 is supplied to the assay strip 1 through the flow channel formed by the opening 33 at the bottom of the second accommodating part 32 and the flow channel forming part 35.

As shown in Fig. 4, a gap (a clearance) C corresponding to the flow channel for the second reagent 46 is formed between a back surface 36 of the flow channel forming part 35 of the multifunctional member 30 and the carrier 2 of the assay strip 1. The gap C is, for example, in the range of 0.01 mm to 1 mm. The second reagent 46 flows out from the opening 33 at the bottom of the second accommodating part 32 toward the carrier 2, and the second reagent 46 that has flowed out flows through the flow channel formed by the gap C and reaches at least above the assay region L1. The second reagent 46 that has reached the assay region L1 infiltrates the assay region L1 from the flow channel.

An absorption pad 6, which will be described later, is disposed at an end part on the downstream side of the assay strip 1. As shown in Fig. 2, in the case body 20, a support part 22 that supports an end part of the assay strip 1 including the absorption pad 6 is formed at a position facing the absorption pad 6. A second accommodating part 32 of the multifunctional member 30 is disposed above the absorption pad 6. The support part 22 also supports the multifunctional member 30 via the absorption pad 6. In addition, in the case body 20, a support part 21 that supports a central part of the assay strip 1 is formed.

<Assay strip>

The assay strip 1 includes a carrier 2, a liquid feeding pad 4, and an absorption pad 6. Then, the carrier 2 is fixedly supported on a back pressure-sensitive adhesive sheet 7.

(Carrier)

The carrier 2 is a porous insoluble carrier for developing a sample, and includes an assay region L1, a control region L2, and a color development region L3. In addition, the carrier 2 includes a label holding pad 3. The label holding pad 3 constitutes a spotting region on which the sample is spotted from dropping port 16. The color development region L3 is disposed on the downstream side of the assay region L1 in a case where the direction toward the assay region L1 with respect to the spotting region is the downstream side of the carrier 2. In the present example, the assay region L1, the control region L2, and the color development region L3 are line-shaped regions extending in a direction perpendicular to the development direction of the sample in the carrier 2.

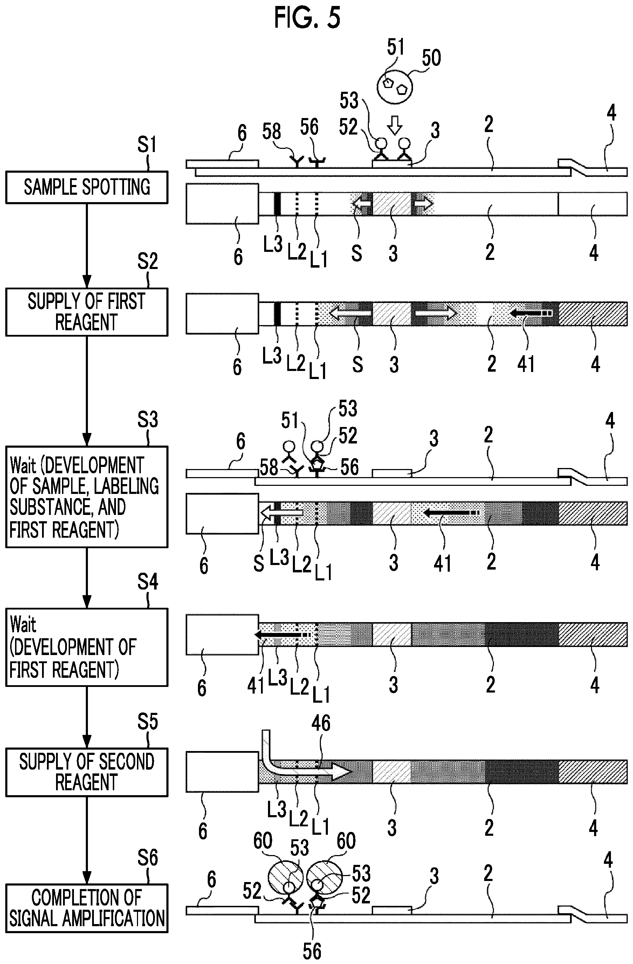

Fig. 2 to Fig. 4 shows a state in which the assay region L1, the control region L2, and the color development region L3 are expressed as lines, but these are not always expressed. Details will be described later, but before developing the sample 50 (see Fig. 5), the first reagent 41 (see Fig. 3 and Fig. 4), and the second reagent 46 (see Fig. 3 and Fig. 4), the colors of the assay region L1 and the control region L2 are substantially the same as the color of the carrier 2 (for example, white), and thus the assay region L1 and the control region L2 cannot be clearly visually recognized at this stage. The assay region L1 is expressed as a line by increasing the color optical density in a case where the sample 50 is developed and the developed sample 50 is positive. Since the color development of the assay region L1 is amplified by silver amplification, which will be described later, the assay region L1 develops a black color.

The control region L2 is also expressed as a line by increasing the color optical density in a case where the sample 50 is developed. Accordingly, the control region L2 is visible. Since the color development of the control region L2 is also subjected to silver amplification, the control region L2 also develops a black color.

On the other hand, only the color development region L3 is expressed and visible as a blackish dark green color (hereinafter, referred to as a dark green color) line even in a stage before the first reagent 41 is developed. However, the color development region L3 is expressed as an orange line by changing a dark green color to an orange color in a case where the first reagent 41 is developed.

As the carrier 2, for example, a porous material such as a nitrocellulose membrane can be used. In addition, the back pressure-sensitive adhesive sheet 7 on which the carrier 2 is fixed is a sheet-shaped substrate having a pressure-sensitive adhesive surface to which the carrier 2 is attached.

(Carrier - label holding pad)

As shown in Fig. 5, a labeling substance 53 is fixed to the label holding pad 3. The labeling substance 53 is modified with the first binding substance 52 that specifically binds to the test substance 51 contained in the sample 50. The label holding pad 3 is fixed on the carrier 2 at a position facing the dropping port 16 (see Fig. 3) of the cover member 10. Therefore, the sample 50 is added dropwise onto the label holding pad 3 from the dropping port 16. Therefore, the label holding pad 3 corresponds to a spotting region on which the sample 50 is spotted.

The label holding pad 3 is fixed at a substantially center position in the longitudinal direction of the carrier 2. As the labeling substance 53, it is possible to use, for example, a gold colloidal particle having a diameter of 50 nm (EM. GC50, manufactured by BBI Solutions). The labeling substance 53 is not limited to the gold colloid, and a metal sulfide that can be used in a general chromatographic method, a coloring particle that are used in an immunoagglutination reaction, or the like can be used, where a metal colloid is particularly preferable. Examples of the metal colloid include a gold colloid, a silver colloid, a platinum colloid, an iron colloid, an aluminum hydroxide colloid, and a composite colloid thereof. In particular, at an appropriate particle diameter, a gold colloid is preferable since it exhibits a red color, a silver colloid is preferable since it exhibits a yellow color, the gold colloid is most preferable among them.

(Carrier - assay region)

As shown in Fig. 5, the assay region L1 includes a second binding substance 56 that specifically binds to the test substance 51 and captures the test substance 51. In the assay region L1, in a case where the test substance 51 is captured by binding the second binding substance 56 to the test substance 51, the first binding substance 52 bonded to the test substance 51 and the labeling substance 53 are captured. In a case where the test substance 51 is included in the sample 50, the test substance 51 and the labeling substance 53 are captured in the assay region L1, and thus the color optical density in the assay region L1 is increased to be not less than a preset reference. The assay region L1 is a region for confirming the presence or absence of the test substance 51 by a labeling signal from the labeling substance 53 captured via the test substance 51.

(Carrier - control region)

The control region L2 includes a third binding substance 58 that specifically binds to the first binding substance 52, and captures the labeling substance 53 via the first binding substance 52. In a case where the sample 50 is spotted on the label holding pad 3, the labeling substance 53 that is not bound to the test substance 51 among the labeling substances 53 modified with the first binding substance 52 is also developed in the carrier 2 toward the assay region L1 together with the sample 50. The labeling substance 53 that is not bound to the test substance 51 passes through the assay region L1 without being captured by the assay region L1. The labeling substance 53 that has passed through the assay region L1 is captured in the control region L2 via the first binding substance 52 by binding the first binding substance 52 to the third binding substance 58. The labeling substance 53 is captured in the control region L2, and thus the color optical density in the control region L2 is increased to be not less than a preset reference. The control region L2 is a region for confirming the completion of the development of the sample 50 by the labeling signal from the labeling substance 53 captured via the first binding substance 52. Therefore, the control region L2 may be referred to as a confirmation region.

(Carrier - color development region)

The color development region L3 contains a substance whose color development state changes in response to the first reagent 41. The color development region L3 indicates that the first reagent 41 has been developed to that region by reacting with the first reagent 41 to develop a color or change a color. For example, in a case where a mixed aqueous solution of an iron nitrate aqueous solution and citric acid (manufactured by Fujifilm Wako Pure Chemical Corporation, 038-06925) is used as the first reagent 41, an aspect in which the color development region L3 is constituted of a color reagent immobilization line on which Bromocresol Green (manufactured by FUJIFILM Wako Pure Chemical Corporation) has been immobilized in a line shape is preferable. This aspect is the aspect of the color development region L3 of the present example. As described above, the color development region L3 of the present example is dark green color before reacting with the first reagent 41, and the dark green color is changed to an orange color in a case where the first reagent 41 reaches the color development region L3. The color development region L3 is sometimes referred to as an amplification index region because the timing of supplying the second reagent 46 after the first reagent 41 is developed is indicated by changing the color development state.

(Binding substance)

The first binding substance 52 that modifies the labeling substance 53 and specifically binds to the test substance 51 is a substance that specifically binds to the test substance, for example, in a case where the test substance is an antigen, an antibody against the antigen, in a case where the test substance is an antibody, an antigen against the antibody, in a case where the test substance is a protein or a low-molecular-weight compound, an aptamer against the protein or the low-molecular-weight compound, or the like.

The second binding substance 56 that is fixed in the assay region L1 and specifically binds to the test substance 51 is a substance that specifically binds to the test substance, for example, in a case where the test substance is an antigen, an antibody against the antigen, in a case where the test substance is an antibody, an antigen against the antibody, in a case where the test substance is a protein or a low-molecular-weight compound, an aptamer against the protein or the low-molecular-weight compound, or the like. The first binding substance 52 and the second binding substance 56 may be the same as or different from each other.

The third binding substance 58 that specifically binds to the first binding substance 52 may be the test substance 51 itself or may be a compound having a site recognized by the first binding substance 52. Examples thereof include a compound obtained by binding a derivative of the test substance 51 to a protein, and the like.

For example, in a case where the test substance 51 is an influenza A virus or a biomarker thereof, anti-influenza A monoclonal antibody (Anti-Influenza A SPTN-5 7307, Medix Biochemica) can be used as the first binding substance 52 and the second binding substance 56, and an anti-mouse IgG antibody (anti-mouse IgG (H+L), rabbit F(ab')2, product number 566-70621, manufactured by FUJIFILM Wako Pure Chemical Corporation) can be used as the third binding substance 58.

(Liquid feeding pad)

The liquid feeding pad 4 is disposed in contact with one end of the carrier 2 and the first reagent 41 is fed to the carrier 2 from the upstream side of the spotting region (constituted of the label holding pad 3). As shown in Fig. 3A, in the liquid feeding pad 4, in a case where the first pressing operation part 11 is pressed, one end of the liquid feeding pad 4 is immersed in the first reagent holding part 40. The liquid feeding pad 4 is formed of a porous material and absorbs the first reagent 41, and the absorbed first reagent 41 is fed to the carrier 2 by a capillary action.

(Absorption pad)

The absorption pad 6 is disposed in contact with the other end of the carrier 2 and absorbs the sample 50, the first reagent 41, and the second reagent 46, which are developed on the carrier 2. The absorption pad 6 is also formed of a porous material.

<Amplifying liquid>

In the present embodiment, the first reagent 41 and the second reagent 46 are amplifying liquids that amplify the color development in the assay region L1 and the control region L2 by reacting with each other. In a case where a metal-based labeling substance such as a gold colloid is used as the labeling substance 53 as in the present example, for example, silver amplification is used as a method of amplifying the labeling signal of the labeling substance 53. The first reagent 41 and the second reagent 46 are, as an example, amplifying liquids used for silver amplification, and the reaction between the first reagent 41 and the second reagent 46 using the labeling substance 53 as a catalyst is an amplification reaction. By the amplification reaction, silver particles having a particle diameter relatively larger than that of the labeling substance 53 are generated.

More specifically, in the present example, the first reagent 41 is a reducing agent that reduces silver ions, and the second reagent 46 is a silver ion. In a case where the first reagent 41, which is a reducing agent, and the second reagent 46, which is a silver ion, are brought into contact with the labeling substance 53, silver particles 60 (see Fig. 5) are generated, and the generated silver particles 60 deposits on the labeling substance 53 using the labeling substance 53 as a nucleus. By depositing the silver particles on the labeling substance 53, silver particles 60 having a particle diameter larger than that of the labeling substance 53 (see Fig. 5) are generated. Accordingly, the labeling signal issued by the labeling substance 53 is amplified, and as a result, the color development of the labeling substance 53 is amplified in the assay region L1 and the control region L2.

(First Reagent)

As the reducing agent as the first reagent 41, any inorganic or organic material or a mixture thereof can be used as long as the silver ion used as the second reagent 46 can be reduced to silver. Preferred examples of the inorganic reducing agent include a reducing metal salt and a reducing metal complex salt, of which the atomic valence is capable of being changed with a metal ion such as $Fe^{2+}$, $V^{2+}$, or $Ti^{3+}$. In a case where an inorganic reducing agent is used, it is necessary to remove or detoxify oxidized ions by complexing or reducing the oxidized ions. For example, in a system in which $Fe^{2+}$ is used as the reducing agent, a complex of $Fe^{3+}$, which is an oxide, is formed using citric acid or ethylenediaminetetraacetic acid (EDTA), which enables the detoxification of the oxidized ions. In the present system, such an inorganic reducing agent is preferably used, and it is more preferable that a metal salt of $Fe^{2+}$ is preferably used.

It is also possible to use a developing agent used in a light-sensitive silver halide photographic material of a wet-type (for example, methyl gallate, hydroquinone, substituted hydroquinone, 3-pyrazolidones, p-aminophenols, p-phenylenediamines, hindered phenols, amidoximes, azines, catechols, pyrogallols, ascorbic acid (or a derivative thereof), and leuco dyes), and other materials obvious to those who are skilled in the related art in the present field, for example, a material described in US6020117A.

As the reducing agent, an ascorbic acid reducing agent is also preferable. The useful ascorbic acid reducing agent includes ascorbic acid, an analogue thereof, an isomer thereof, and a derivative thereof. Preferred examples thereof include D- or L-ascorbic acid and a sugar derivative thereof (for example, $\gamma$-lactoascorbic acid, glucoascorbic acid, fucoascorbic acid, glucoheptoascorbic acid, or maltoascorbic acid), a sodium salt of ascorbic acid, a potassium salt of ascorbic acid, isoascorbic acid (or L-erythroascorbic acid), a salt thereof (for example, an alkali metal salt, an ammonium salt, or a salt known in the related technical field), ascorbic acid of the enediol type, ascorbic acid of the enaminol type, ascorbic acid of the thioenol type. Particularly, D-, L-, or D,L-ascorbic acid (and an alkali metal salt thereof) or isoascorbic acid (or an alkali metal salt thereof) is preferable, and a sodium salt is a preferred salt. A mixture of these reducing agents can be used as necessary.

(Second reagent)

The solution containing silver ions, which is used as the second reagent 46, is preferably a solution obtained by dissolving a silver ion-containing compound in a solvent. As the silver ion-containing compound, an organic silver salt, an inorganic silver salt, or a silver complex can be used. An inorganic silver salt or a silver complex is preferable. As the inorganic silver salt, it is possible to use a silver ion-containing compound having a high solubility in solvents such as water, and examples thereof include silver nitrate, silver acetate, silver lactate, silver butyrate, and silver thiosulfate. Silver nitrate is particularly preferable. The silver complex is preferably a silver complex in which silver is coordinated with a ligand having a water-soluble group such as a hydroxyl group or a sulfone group, and examples thereof include silver hydroxythioether.

<Immunochromatographic method>

An immunochromatographic method will be described with reference to Fig. 5. Here, a case where the sample 50 includes the test substance 51, that is, on the premise that the sample 50 is positive will be described.

First, the sample 50 is spotted on the label holding pad 3 which is the spotting region (Step S1). The test substance 51 in the sample 50, which is spotted on the label holding pad 3, specifically binds to the first binding substance 52 that modifies the labeling substance 53 contained in the label holding pad 3. The sample 50 is developed on the downstream side from the label holding pad 3 in the carrier 2 by the capillary action in the carrier 2. Apart of the sample 50 is also developed on the upstream side.

Next, the first reagent 41 is supplied (Step S2). The first reagent 41 is supplied from the liquid feeding pad 4. The first reagent 41 is supplied to the carrier 2 via the liquid feeding pad 4 and is developed on the downstream side.

After that, the process waits until the first reagent 41 is developed on the downstream side (Step S3 and Step S4). "Wait" shown in Fig. 5 means an action of waiting. The first reagent 41 is gradually developed to the downstream side, and the sample 50 to be developed from the label holding pad 3 and the labeling substance 53 modified with the first binding substance 52 are developed to the downstream side to be pushed by the first reagent 41 (Step S3).

The test substance 51 in the sample 50 that has been developed to the downstream side and has reached the assay region L1 is captured by the second binding substance 56 of the assay region L1. That is, the labeling substance 53 is captured in the assay region L1 via the test substance 51 and the first binding substance 52. On the other hand, the labeling substance 53 that is not bound to the test substance 51 passes through the assay region L1 without being captured and is captured by the third binding substance 58 of the control region L2.

In a case where the development of the first reagent 41 proceeds and the first reagent 41 reaches the color development region L3 (Step S4), the color development region L3 reacts with the first reagent 41 to change the color development state. In the present example, the color development region L3 is dark green color before reacting with the first reagent 41, and the dark green color is changed to an orange color by reacting with the first reagent 41.

After the first reagent 41 is sufficiently developed, the second reagent 46 is supplied to the carrier 2 (Step S5). The second reagent 46 is supplied to the carrier 2 from the downstream side of the color development region L3 and is developed on the upstream side. Here, the first reagent 41 is a first amplifying liquid containing a reducing agent that reduces silver ions, and the second reagent 46 is a second amplifying liquid containing silver ions. By reacting the first amplifying liquid with the second amplifying liquid, the silver particles 60 are generated using the gold colloidal particles that are the labeling substance 53 as a catalyst. Accordingly, the labeling signal is amplified (Step S6).

<Immunochromatographic assay apparatus>

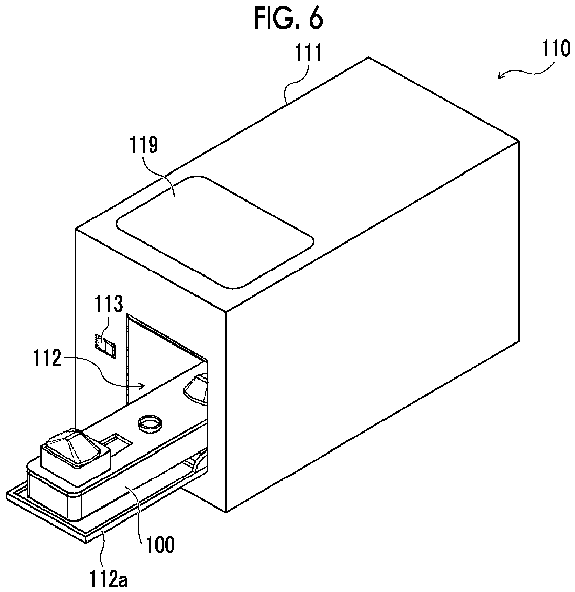

As described above, in the cartridge 100, it is possible for the user to visually confirm the observation window 18 to confirm the color development state of the assay region L1. Further, in the cartridge 100, it is also possible to confirm the color development state of the assay region L1 by using an immunochromatographic assay apparatus 110 (hereinafter, simply referred to as an assay apparatus 110) as shown in Fig. 6. The assay apparatus 110 detects a color development state of the assay region L1 of the loaded cartridge 100, determines whether the sample is positive or negative based on the detected color development state, and presents the determination result.

As shown in Fig. 6, the assay apparatus 110 includes a case body 111, and the case body 111 includes a cartridge loading part 112 in which the cartridge 100 is attachably and detachably loaded. As an example, an opening for inserting the cartridge 100 into the case body 111 and an opening and closing lid 112a for opening and closing the opening are provided on the front surface of the case body 111. The opening and closing lid 112a is opened when the cartridge 100 is loaded, the cartridge 100 is inserted into the case body 111, and the opening and closing lid 112a is closed after the cartridge 100 has been loaded into the cartridge loading part 112. The assay is performed in a state where the opening and closing lid 112a is closed.

The assay apparatus 110 of the present example, as an example, a cartridge 100 is loaded in a state in which the spotting of the sample on the carrier 2 and the supply of the first reagent 41 and the second reagent 46 to the carrier 2 is started. That is, in the present example, the pressing operation of the first pressing operation part 11 and the second pressing operation part 12 is performed by the user before the cartridge 100 is loaded into the assay apparatus 110. Accordingly, the cartridge 100 is in the state shown in Fig. 3B, and the cartridge 100 is loaded into the assay apparatus 110 in this state. The assay apparatus 110 detects a color development state of the assay region L1 of the loaded cartridge 100, and presents the result of whether the sample is positive or negative. In a case of where a plurality of samples are assayed, the cartridge 100 for each sample is loaded one by one into the assay apparatus 110.

In addition, a power switch 113 is provided on the front surface of the case body 111, and a monitor 119 is provided on the upper surface of the case body 111. A determination result, an error message, and the like are displayed on the monitor 119. As an example, the monitor 119 is a touch panel monitor, and various operation screens are displayed. Through the operation screen, the user can input a start instruction of processing and an operation instruction such as selection of an assay procedure.

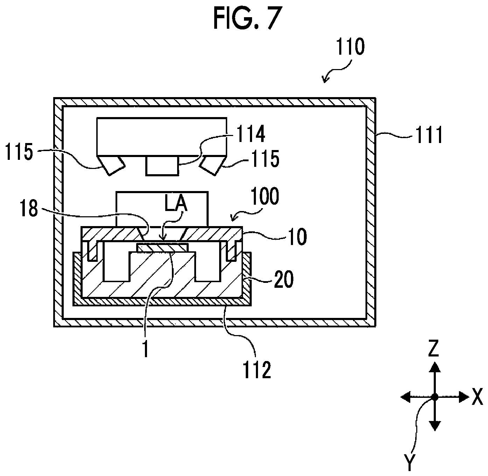

As shown in Fig. 7 and Fig. 8, a detection unit 114 and an illumination unit 115 are provided in the assay apparatus 110.

(Detection unit)

The detection unit 114 optically detects the color development state of the assay region L1, the control region L2, and the color development region L3, and outputs a detection signal indicating the color development state to the processor (not shown). The detection unit 114 is, for example, an image sensor such as a complementary metal oxide semiconductor (CMOS) image sensor and a charge coupled device (CCD) image sensor and images the observation region LA including the assay region L1, the control region L2, and the color development region L3. The detection unit 114 of the present example outputs the captured image including the observation region LA to the processor as a detection signal.

The detection unit 114 is disposed at a directly-facing position directly facing the observation region LA of the cartridge 100 in a state where the cartridge 100 is loaded in the assay apparatus 110. The directly-facing position is a position facing the observation region LA, and more specifically, the facing position is a position facing in a posture where the imaging surface of the detection unit 114 and a surface of the observation region LA are parallel on the front of the observation window 18 exposing the observation region LA.

(Illumination unit)

The illumination unit 115 is an example of a light source that illuminates the observation region LA. The illumination unit 115 has, for example, a semiconductor light source such as a light emitting diode as a light emitting element (not shown) that emits illumination light. An illumination window (not shown) that emits illumination light is provided at one end part of the illumination unit 115, and the light emitting element is disposed inside the illumination window. There are, for example, two illumination units 115, which are disposed on both sides of the detection unit 114. More specifically, the illumination unit 115 are disposed at positions symmetrical with respect to the illumination unit 115 in the X direction. Since the detection unit 114 is disposed at a directly-facing position to the observation region LA, the two illumination units 115 located on both sides of the detection unit 114 are irradiated with illumination light from an oblique direction with respect to the observation region LA. The observation region LA is exposed from the observation window 18 which is an opening portion. The illumination unit 115 illuminates the observation region LA through the observation window 18.

The detection unit 114 images the observation region LA in a state of being illuminated by the illumination unit 115. The processor acquires the captured image from the detection unit 114, and trims the observation image corresponding to the observation region LA from the acquired captured image. Then, the processor detects a color development state of the assay region L1 based on the observation image to determine whether the sample is positive or negative.

(Assay mode)

The assay apparatus 110 is capable of selecting two types of assay modes. The first mode is a mode in which the cartridge 100 is loaded into the assay apparatus 110 after the user has pressed both the first pressing operation part 11 and the second pressing operation part 12. In addition, the second mode is a mode in which the cartridge 100 in which the user has pressed only the first pressing operation part 11 is loaded into the assay apparatus 110, and a mode in which the second pressing operation part 12 is operated by the internal mechanism of the assay apparatus 110. In any mode selected, the detection unit 114 images the observation region LA in a state in which the second pressing operation part 12 is operated and the second reagent 46 is supplied.

<Observation window>

As shown in Fig. 9, the observation window 18 has a rectangular shape having a short side in the X direction and a long side in the Y direction. Since the Y direction is the longitudinal direction of the cartridge 100, that is, the observation window 18 has a rectangular shape in which the longitudinal direction (Y direction) is along the longitudinal direction of the cartridge 100. The illumination unit 115 illuminates the observation region LA from the X direction intersecting the Y direction, which is the longitudinal direction of the observation window 18.

In the observation region LA, the assay region L1, the control region L2, and the color development region L3 are disposed along a direction (X direction) intersecting the longitudinal direction of the observation window 18.

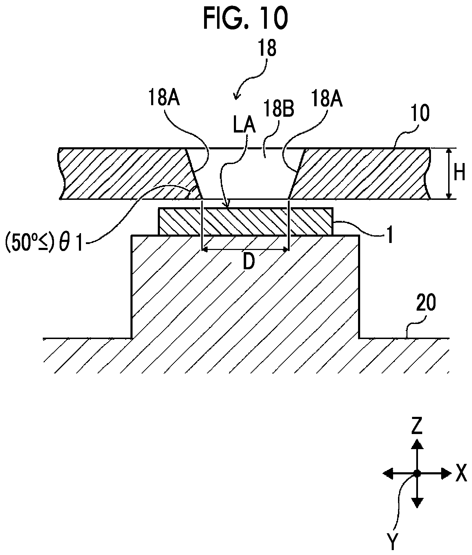

As shown in Fig. 10, in the observation window 18, the two inner peripheral walls 18A along the longitudinal direction and the two inner peripheral walls 18B along the lateral direction (X direction) are formed inclinedly with respect to the surface of the observation region LA in the carrier 2. In addition, the inclined angle $\theta 1$ is 50° or more. The "inclined angle $\theta 1$" is an angle with respect to the surface of the observation region LA, and is 0° in a case where it is parallel to the surface of the observation region LA and 90° in a case where it is perpendicular to the surface. "Inclination" includes an angle perpendicular to the surface of the observation region LA.

In addition, the inner peripheral walls 18A and 18B are processed surface having been subjected to the roughening treatment (see the hatched regions in Fig. 8). That is, the inner peripheral wall of the observation window 18 is subjected to a roughening treatment over the entire circumference. This processed surface is formed by emboss processing as an example, and as the surface roughness, the arithmetic average height Sa is 100 $\mu$m or less among the parameters related to the surface roughness defined in the international standard (ISO25178).

As shown in Fig. 10, in a case where an opening width in a direction along the surface of the observation region LA in the carrier 2 and in a short side direction (X direction) of the observation window 18 is D, and a height of the inner peripheral wall 18A in a direction (Z direction) orthogonal to the opening width is H, $0.5D \leq H \leq 1.5D$.

<Action and effect>

As shown in Fig. 3, the cartridge 100 of the present disclosure includes an assay strip 1 including a carrier 2 having an assay region L1, and a case 9 in which the assay strip 1 is accommodated. In addition, the cartridge 100 includes the observation window 18 as an opening portion that is provided in the case 9, is used for observing the assay region L1, and is illuminated by the illumination unit 115 in the assay apparatus 110 as en example of a light source as shown in Fig. 7 and Fig. 8.

As shown in Fig. 10, in at least a part of the inner peripheral wall of the observation window 18 (in the present embodiment, the inner peripheral walls 18A and 18B), the inclined angle $\theta 1$, which is an angle with respect to the surface of the observation region LA having the assay region L1 in the carrier 2 is 50° or more. Furthermore, the inner peripheral walls 18A and 18B are processed surfaces that have been subjected to a roughening treatment.

With such a configuration, it is possible to provide the cartridge 100 in which the light quantity distribution of the observation region LA including the assay region L1 and the peripheral region thereof is likely to be even in a case where the observation window 18 is illuminated, as compared with the prior art.

That is, as shown in Fig. 7 and Fig. 8, in the assay apparatus 110, in addition to the illumination unit 115 which is an example of the light source that illuminates the observation window 18, a detection unit 114 that optically detects the color development state of the assay region L1 is provided. Therefore, the more the light quantity distribution of the illumination light is uneven in the observation region LA including the assay region L1 and the peripheral region thereof, the higher the possibility of erroneous detection is.

Here, in a case where the illumination unit 115 can be disposed at a directly-facing position directly facing the observation region LA including the assay region L1 as the detection unit 114, the observation region LA can be irradiated with the illumination light from the front. In this case, since the distance from the illumination unit 115 is relatively even in the entire region of the observation region LA, it is easy to ensure the evenness of the light quantity distribution.

However, as shown in Fig. 7 and Fig. 8, the necessity of disposing the detection unit 114 at a directly-facing position to the observation region LA is high, and in such a case, the illumination unit 115 must be disposed on the side of the detection unit 114. Therefore, the illumination light is emitted from an oblique direction, not from the front of the observation region LA.

In this case, as compared with a case where the illumination unit 115 is disposed at the directly-facing position, the distance from the illumination unit 115 is likely to be biased depending on the location of the observation region LA and the illumination light is eclipsed in the periphery of the observation window 18, and thus a part of the observation region LA is likely to be shadowed. In this case, the light quantity distribution in the observation region LA tends to be uneven.

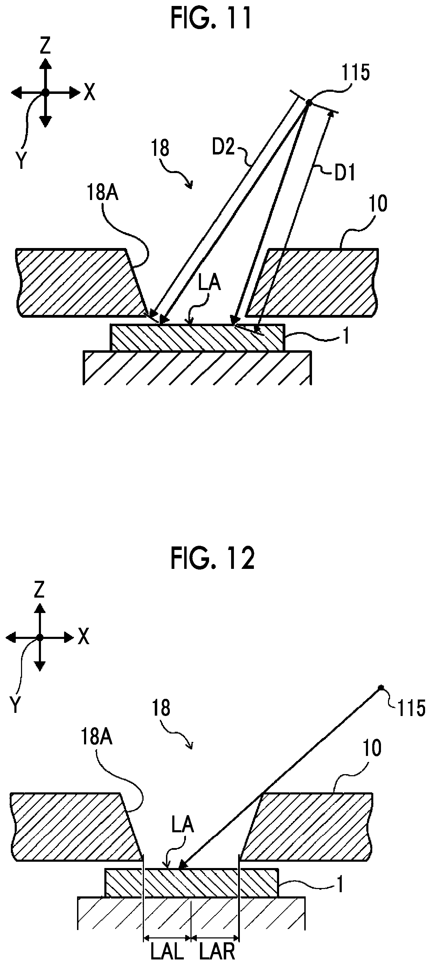

For example, a case where the observation window 18 is illuminated from the illumination unit 115 disposed obliquely upward at right side of the observation window 18 as shown in Fig. 11 is considered. In this case, since a difference occurs in the distance from the illumination unit 115 between the right side and the left side of the observation region LA, the light quantity distribution tends to be uneven. That is, since the distance D2 from the illumination unit 115 to the left side portion of the observation region LA is longer than the distance D1 to the right side portion, the left side portion is darker.

In addition, as shown in Fig. 12, since the illumination light is incident from obliquely upward at right side, the illumination light is likely to be directly incident into an observation region LAL located on the left side of the observation window 18 without being eclipsed, but since there is a possibility that the illumination light is eclipsed in the vicinity of the right side of the observation window 18, the light quantity of the illumination light directly incident on the observation region LAR located on the right side of the observation window 18 is relatively small and tends to be shadowed. In this way, in a case where the observation window 18 is irradiated with the illumination light from an oblique direction, the light quantity distribution in the observation region LA tends to be uneven.

The illumination unit 115 shown in Fig. 11 and Fig. 12 is different in position in the assay apparatus 110 from the illumination unit 115 shown in Fig. 8, but these show examples of the position of the illumination unit 115. In the assay apparatus 110, the illumination unit 115 may be disposed at various positions. For example, in a case where the plane dimension of the detection unit 114 is large, the illumination unit 115 may be disposed at a position farther from the observation window 18 than in a case where the plane dimension is small. In addition, in a case where the vertical dimension of the inner region of the assay apparatus 110 is small, the illumination unit 115 may be disposed at a lower position.

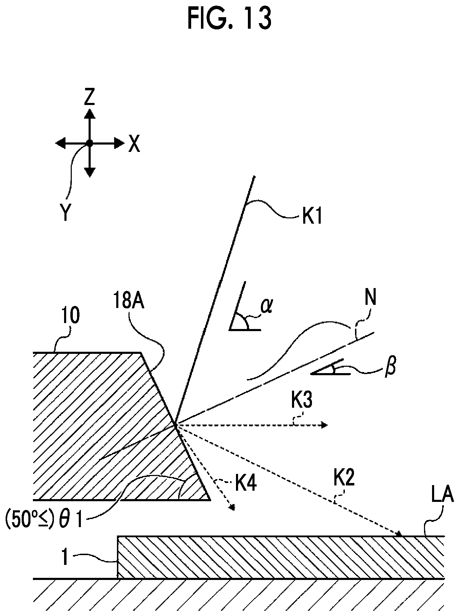

According to the cartridge 100 of the present disclosure, as shown in Fig. 13, at least a part of the inner peripheral wall 18A of the observation window 18 has an inclined angle $\theta 1$ with respect to the surface of the observation region LA of 50° or more, and thus the illumination light incident on the inner peripheral wall 18A of the observation window 18 from an oblique direction is more likely to be reflected toward the observation region LA as compared with a case where the angle $\theta 1$ is less than 50°.

For example, the illumination light directed from the obliquely upward at right side toward the observation window 18 is incident on the inner peripheral wall 18A on the left side of the observation window 18, as shown by the straight line K1 as an example. In a case where the angle $\theta 1$ of the processed surface of the inner peripheral wall 18A is 50° or more, the incident illumination light is more likely to be reflected toward the right side of the observation region LA as shown by the arrow K2 than in a case where the angle $\theta 1$ is less than 50°. Accordingly, the light quantity on the right side of the observation region LA can be increased.

Furthermore, since at least a part of the inner peripheral wall 18A is subjected to the roughening treatment, the scattering of the reflected light is large as compared with the case of not being subjected to the roughening treatment. Therefore, the unevenness of the light quantity distribution due to the difference in the distance from the illumination unit 115 (see Fig. 11) is suppressed by the light scattering effect.

In a case where the illumination unit 115 is a light emitting element such as a light emitting diode, the illumination unit 115 emits divergent light that spreads radially with the light emitting point as a base point. In Fig. 13, an axis passing through the center of the luminous flux of the divergent light is shown by a straight line K1 as the central axis of the luminous flux. In addition, the arrow K2 is an arrow drawn in linear symmetry on the straight line K1 with the normal line N of the inner peripheral wall 18A as the "target axis", and shows an example of the direction in which the reflected light, which is the scattered light, is directed. Furthermore, the arrows K3 and K4 indicate another example of the direction in which the reflected light is directed. In this way, the reflected light of the inner peripheral wall 18A is diffused in various directions. Due to such a mechanism by which the reflected light is diffused, the light quantity distribution in the observation region LA uniform is more likely to be even as compared with the prior art.

Here, among the reflected light which is a illumination light reflected by the inner peripheral wall 18A of the observation window 18, the reflected light directly directed to the detection unit 114 (see Fig. 7 and Fig. 8) without passing through the observation region LA is unnecessary light that causes noise for the detection unit 114 because it does not have the information of the observation region LA.

In the present embodiment, since the inclined angle θ1 of the inner peripheral wall 18A of the observation window 18 is 50° or more, the reflected light quantity directed to the observation region LA of the illumination light reflected by the inner peripheral wall 18A is increased as compared with a case where the inclined angle θ1 is less than 50°. Accordingly, unnecessary light directly directed to the detection unit 114 can be relatively reduced. Since unnecessary light that causes noise is reduced, more accurate assay is possible.

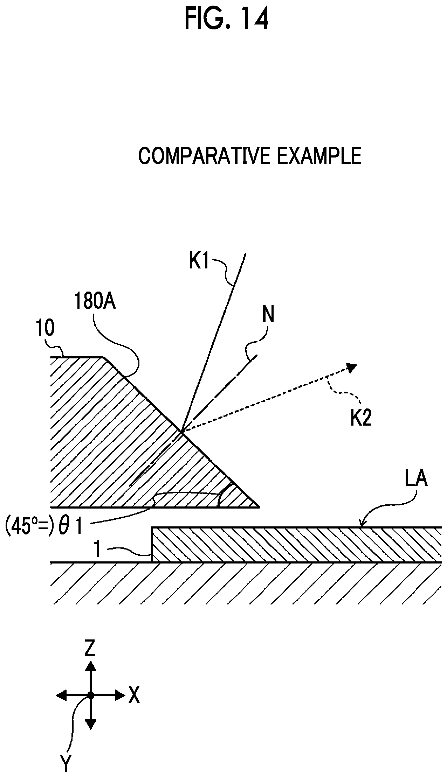

Note that Fig. 14 shows the inner peripheral wall 180A as a comparative example in which the angle θ1 with respect to the surface of the observation region LA is less than 50°, for example, 45°. In the present example, in the reflected light of the incident light indicated by the straight line K1, the reflected light indicated by the arrow K2 reflected at the angle in linear symmetry on the straight line K1 with the normal line N of the inner peripheral wall 18A as the target axis is directed upward, not to the observation region LA. In a case where the angle θ1 with respect to the surface of the observation region LA is less than 50° as in this inner peripheral wall 180A, it is difficult to reduce unnecessary light directly directed to the detection unit 114 as compared with a case where the angle θ1 is 50° or more.

In addition, according to the cartridge 100 of the present disclosure, as shown in Fig. 9, the observation window 18 has a rectangular shape. Then, of the inner peripheral walls 18A and 18B, the processed surface subjected to roughening treatment on at least the inner peripheral wall 18A extending in the longitudinal direction is formed (in the present example, the inner peripheral wall 18B is also a processed surface). In a case where the observation window 18 has a rectangular shape, the illumination unit 115 is often disposed such that the longitudinal direction (Y direction) having a wide frontage intersects the illumination direction to secure the incident light quantity. For example, in the present embodiment, as shown in Fig. 8, the illumination unit 115 is disposed to illuminate the observation window 18 from a direction (X direction) orthogonal to the longitudinal direction of the observation window 18. Therefore, in a case where the processed surface is formed on the inner peripheral wall 18A which is the wall surface extending in the longitudinal direction, the effect of suppressing the unevenness of the light quantity distribution is large.

In the cartridge 100 of the present disclosure, the processed surface subjected to rough surface treatment is also formed on the inner peripheral wall 18B extending in the lateral direction, and the processed surface is formed over the entire circumference (both the inner peripheral walls 18A and 18B in the present example) of the inner peripheral wall of the observation window 18 as an example of the opening portion.

In a case where only the specific wall surface, for example, only the inner peripheral wall 18A is subjected to the roughening treatment, it is difficult to obtain the scattering effect of the reflected light of the light incident on the inner peripheral wall 18B which is the other wall surface. However, as in the present disclosure, in a case where the observation window 18 is subjected to the roughening treatment over the entire circumference of the inner peripheral wall, the area of the wall surface where the scattering effect can be obtained is large, and thus the scattering effect of the reflected light can be enhanced.

For example, in a case where the observation window 18 has a rectangular shape as in the present embodiment, the illumination light incident on not only the inner peripheral wall 18A extending in the longitudinal direction but also the inner peripheral wall 18B extending in the lateral direction can be scattered. A part of the light scattered by the inner peripheral wall 18B irradiates the observation region LA. Therefore, it is easy to suppress the unevenness of the light quantity distribution over the entire surface of the observation region LA.

In the present embodiment, the processed surface is formed over the entire circumference of the inner peripheral wall of the observation window 18, but the embodiment of the present disclosure is not limited to this. For example, the processed surface may be formed only on the entire region of the inner peripheral wall 18A. In addition, the processed surface may be formed only at a position overlapping the assay region L1 and a peripheral portion of the position of the inner peripheral wall 18A.

In addition, in the cartridge 100 of the present disclosure, a line-shaped assay region L1 is disposed along a direction (X direction) intersecting the longitudinal direction of the observation window 18. Therefore, in a direction in which the assay region L1 extends, it is possible to obtain an effect of suppressing the unevenness of the light quantity distribution due to the formation of the processed surface on the inner peripheral wall 18A extending in the longitudinal direction.

That is, since the processed surface is formed on the inner peripheral wall 18A extending in the Y direction, which is the longitudinal direction, the unevenness of the light quantity distribution in the X direction of the observation region LA can be suppressed, and thus the assay region L1 is easy to be illuminated evenly.

In addition, in the cartridge 100 of the present disclosure, the surface roughness of the processed surface has an arithmetic average height Sa of 100 $\mu$m or less among the parameters related to the surface roughness defined by the international standard (ISO25178). Accordingly, as compared with a case where the arithmetic average height Sa is larger than 100 $\mu$m, the reflected light is likely to be diffused at a wide angle, and the effect of suppressing the unevenness of the light quantity distribution can be enhanced.

In addition, in the cartridge 100 of the present disclosure, the processed surface is subjected to roughening treatment by the emboss processing. Therefore, the durability of the roughening treatment is higher as compared with a case where, for example, a sheet material having been roughened is attached.

In addition, the roughening treatment in the present disclosure includes attaching a roughened sheet material or the like in addition to roughening by forming regular or irregular roughness. Examples of the regular roughness include roughness having a serrated cross section and a constant pitch, roughness formed by two-dimensionally arranging a plurality of circular or quadrangular convex portions at regular intervals, and the like.

In addition, examples of the irregular roughness include chemical treatment by etching and emboss processing by sandblasting or the like. Furthermore, the mold for molding the cover member 10 of the case 9 may be subjected to the roughening treatment by etching or sandblasting. In a case where the mold is roughened, the roughness of the mold is transferred to the cover member 10.

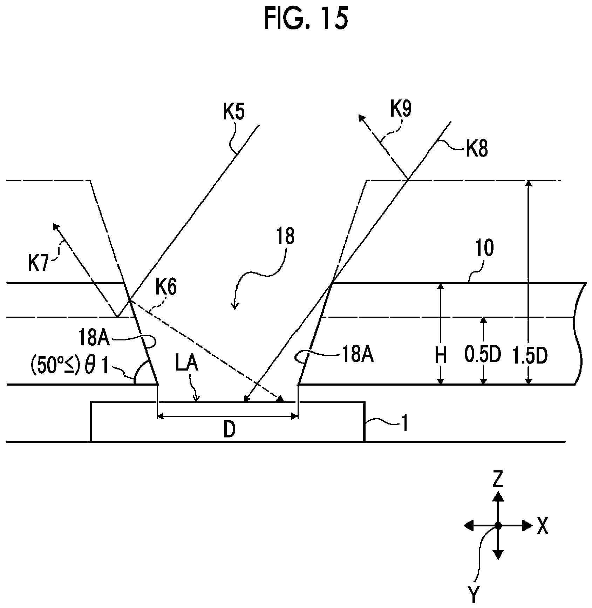

In addition, in the cartridge 100 of the present disclosure, as shown in Fig. 10, the height H of the inner peripheral wall 18A of the observation window 18 is 0.5 times or more the opening width in a direction along the surface of the observation region LA and in the short side direction of the observation window 18. Accordingly, as shown in Fig. 15, as compared with a case where the height H of the inner peripheral wall 18A is less than 0.5 times the opening width D of the observation window 18 (an example of the opening portion), the area by which the light can be reflected is large, and thus the scattering effect of the light incident on the observation window 18 due to the reflection on the inner peripheral wall is enhanced.

Specifically, in a case where the height H of the inner peripheral wall 18A is 0.5 times "or more" the opening width D of the observation window 18, the light indicated by the straight line K5 is incident on the inner peripheral wall 18A and reflected toward the observation region LA as indicated by the arrow K6. On the other hand, in a case where the height H of the inner peripheral wall 18A is "less than" 0.5 times the opening width D of the observation window 18, the light indicated by the straight line K5 is not incident on the inner peripheral wall 18A and is incident on the upper surface of cover member 10 and reflected upward as indicated by the arrow K7.

In addition, in the cartridge 100 of the present disclosure, the height H of the inner peripheral wall 18A is 1.5 times or less the opening width D of the observation window 18. Accordingly, as compared with a case where the height H of the inner peripheral wall 18A is larger than 1.5 times the opening width D of the observation window 18, it is difficult to block the illumination light and thus the illumination light easily enters the observation region LA.

Specifically, in a case where the height H of the inner peripheral wall 18A is 1.5 times "or less" the opening width D of the observation window 18, the light indicated by the arrow K8 is incident on the observation region LA. On the other hand, in a case where the height H of the inner peripheral wall 18A is "larger than" 1.5 times the opening width D of the observation window 18, the light indicated by the arrow K8 is not incident on the observation region LA, and is incident on the upper surface of the cover member 10 and reflected upward as indicated by the arrow K9.

In addition, in the cartridge 100 of the present disclosure, as shown in Fig. 1, Fig. 2, and Fig. 8, a structure having a convex shape in a direction away from the surface of the observation region LA is provided around the observation window 18. Such a structure includes a boss that forms a dropping port 16 for adding dropwise a sample on an assay strip 1 disposed inside the cartridge 100. In addition, as another example, a pressing unit (for example, a second pressing operation part 12) for developing the amplifying liquid on the assay strip 1 is included.

In the cartridge 100, at least a part of the outer peripheral wall of these structures may be subjected to the roughening treatment. Such a place is subjected to roughening treatment and thus a part of the scattered light reflected by the outer peripheral wall can be incident on the observation region LA. Accordingly, the light quantity in the observation region LA can be increased.

In the cartridge 100 of the present disclosure, as shown in Fig. 10 and Fig. 13, the angle $\theta 1$ of the inner peripheral wall 18A with respect to the surface of the observation region LA is 50° or more. This inclined angle $\theta 1$ is more preferably 70° or more and 90° or less. In a case where the inclined angle $\theta 1$ is less than 70°, the light quantity of the light reflected toward the observation region LA is smaller as compared with a case where the inclined angle $\theta 1$ is 70° or more. In addition, in a case where the inclined angle $\theta 1$ is larger than 90°, it is difficult for light to be incident on the observation window 18, and thus the light quantity incident on the observation region LA is reduced.

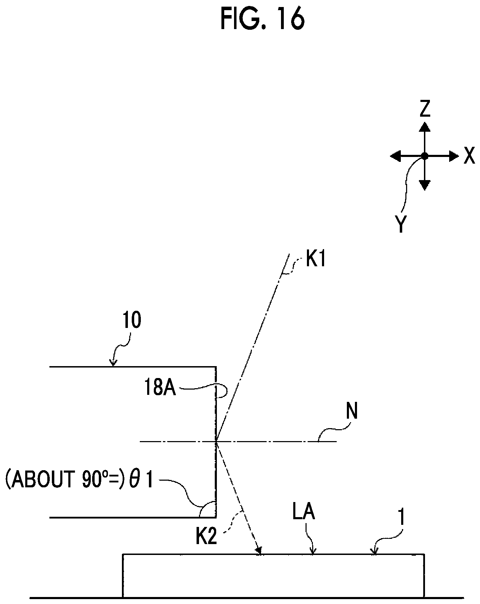

As an example of the inclined angle $\theta 1$ included in the range of 70° or more and 90° or less, Fig. 16 shows an example in which the inclined angle $\theta 1$ is about 90°. In this way, by increasing the angle $\theta 1$ in a range of 70° or more and 90° or less, the light incident on the observation window 18 is more likely to be reflected downward.

Accordingly, the unevenness of the light quantity distribution in the observation region LA can be further suppressed. In a case where the cover member 10 is molded by injection molding or the like using a mold, a draft of the mold is required and thus the inclined angle θ1 is slightly smaller than 90° as strictly.

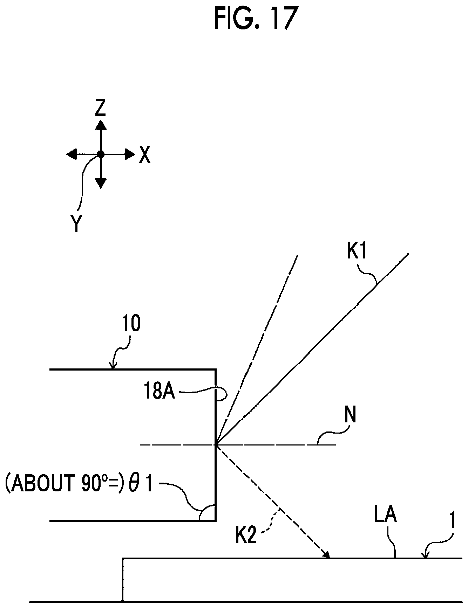

As shown in Fig. 17, the larger the inclined angle θ1 is, the smaller the incidence angle of the light directed from the illumination unit 115 toward the observation window 18 is. Similarly to the inclined angle θ1, the incidence angle is 0° in a case of being parallel to the surface of the observation region LA and 90° in a case of being perpendicular to the surface. By reducing the incidence angle, the position of the illumination unit 115 can be lowered. Accordingly, the assay apparatus 110 for loading the cartridge 100 can be miniaturized. The inclined angle θ1 is set in consideration of a plurality of such factors.

In addition, in a case where the cartridge 100 is loaded in the assay apparatus 110, the relative positional relationship between the illumination unit 115 and the observation window 18 is preferably as follows.

That is, as shown in Fig. 13, a straight line K1 indicating the axis passing through the center of the luminous flux of the divergent light emitted from the illumination unit 115 (see Fig. 7 and Fig. 8) as the central axis of the luminous flux and a normal line N of the inner peripheral wall 18A of the observation window 18 preferably satisfy the following relationship.

In a case where the angle between the surface of the observation region LA in the assay strip 1 and the straight line K1 is defined as a first angle α, and the angle between the surface of the observation region LA and the normal line N of the inner peripheral wall 18A is a second angle β, the condition that β < α and α are 90° or less is satisfied.

In a case where the assay apparatus 110 in which the illumination unit 115 is disposed to satisfy this condition is used, it is easy to increase the light quantity of the reflected light directed to the observation region LA among the reflected light reflected by the inner peripheral wall 18A.

(Modified example of inner peripheral wall)

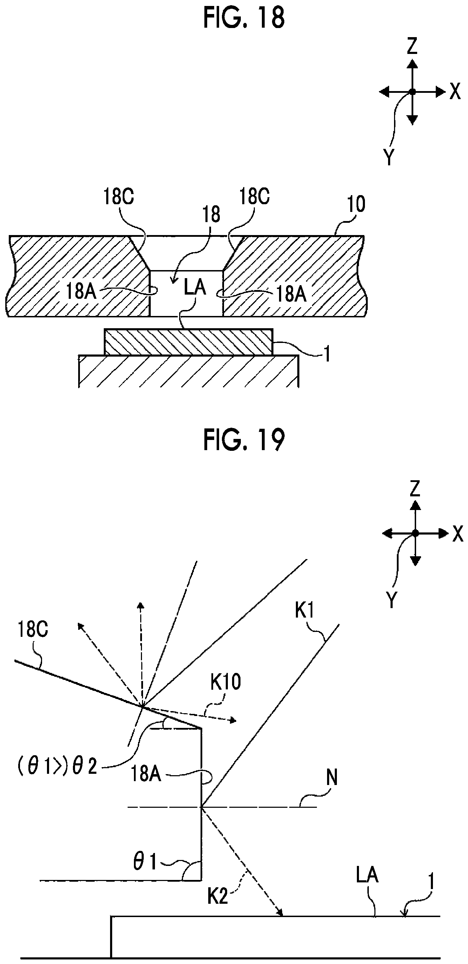

In addition, the shape of the inner peripheral wall can be deformed as follows. In the example shown in Fig. 18, in a case where the inner peripheral wall 18A is the first inner peripheral wall, around the observation window 18, the inner peripheral wall 18C is formed as the second inner peripheral wall that rises in a direction away from the surface of the observation region LA along the intersection direction intersecting the surface of the observation region LA. This inner peripheral wall 18C rises from an end edge of the inner peripheral wall 18A.

Such an aspect is an aspect in which a recess that drops on inner side in which the assay strip 1 is disposed is formed in a part of the cover member 10 and the observation window 18 is formed at the bottom of the recess. Since the recess is formed, the inner peripheral wall of the recess is the inner peripheral wall 18C. By forming the recess, the observation window 18 can be easily brought close to the observation region LA even in a case where the maximum thickness of the cover member 10 is large. Since the observation window 18 can be brought close to the observation region LA, the light quantity incident on the observation window 18 can be incident on the observation region LA without waste as compared with a case where the observation window 18 and the observation region LA are separated from each other.

In addition, at least a part of the inner peripheral wall 18C is preferably subjected to a roughening treatment similarly to the inner peripheral wall 18A. Accordingly, as shown by an arrow K10 or the like in Fig. 19, the reflected light reflected from the inner peripheral wall 18C can be diffused. Therefore, the light quantity distribution is more likely to be even as compared with a case where the inner peripheral wall 18C is not subjected to the roughening treatment.

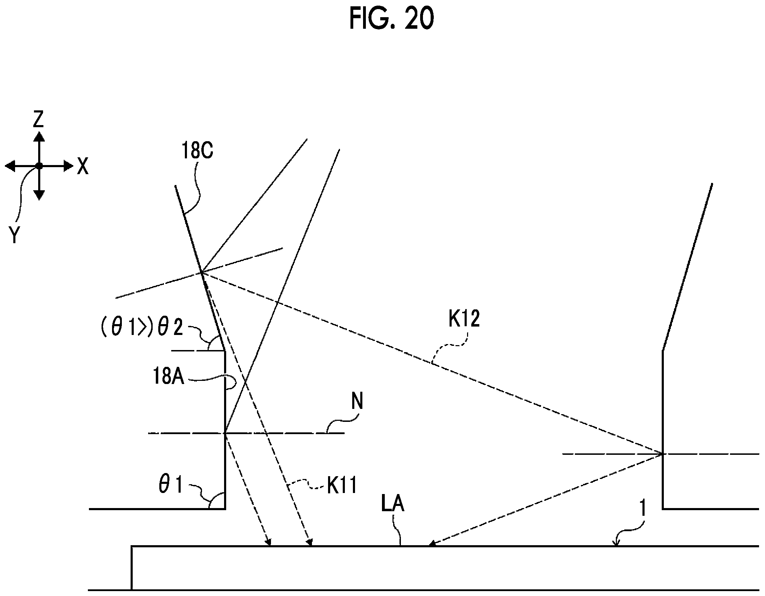

In addition, in a case where the inner peripheral wall 18C is formed as the second inner peripheral wall, as shown in Fig. 19 and Fig. 20, the inclined angle θ2 of the inner peripheral wall 18C with respect to the surface of the observation region LA is preferably smaller than the inclined angle θ1 of the inner peripheral wall 18A with respect to the surface of the observation region LA.

Accordingly, as shown in Fig. 20, the light reflected by the inner peripheral wall 18C is more likely to be directed upward than the light reflected by the inner peripheral wall 18A. Therefore, the light reflected by the inner peripheral wall 18C is likely to be incident not only on the observation region LA as indicated by the arrow K11 but also on the inner peripheral wall 18A as indicated by the arrow K12. Since light is incident on the observation region LA through various paths such as light passing through two reflecting surfaces of the inner peripheral wall 18C and the inner peripheral wall 18A, a synergistic effect with the light scattering effect by the inner peripheral wall 18A can be obtained.

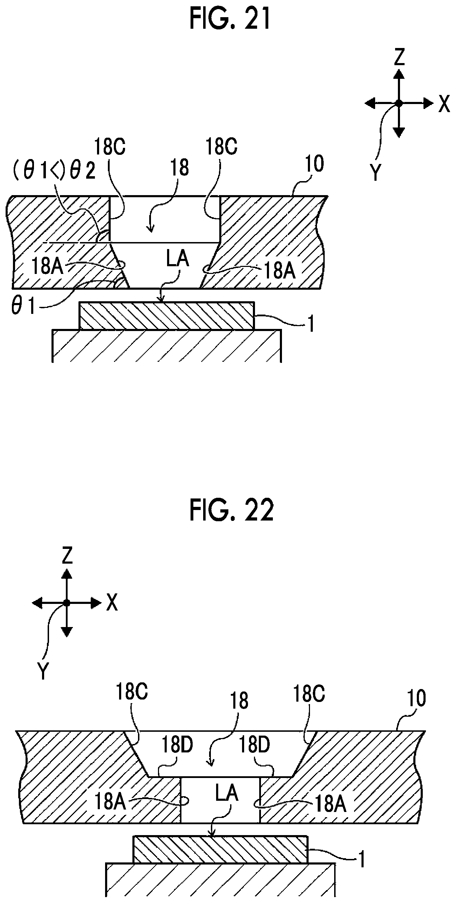

For example, as shown in Fig. 21, the inclined angle θ2 of the inner peripheral wall 18C with respect to the surface of the observation region LA may be larger than the inclined angle θ1 of the inner peripheral wall 18A with respect to the surface of the observation region LA.

In addition, in a case of forming the inner peripheral wall 18C as the second inner peripheral wall, as shown in Fig. 22, a plane portion 18D may be provided between the inner peripheral wall 18A and the inner peripheral wall 18C. The plane portion 18D is a plane portion that is provided around the observation window 18 and extends in a direction along the surface of the observation region LA. The inner peripheral wall 18C rises from the end edge of the plane portion 18D opposite to the observation window 18. By providing such a plane portion, the thickness of the peripheral portion of the observation window 18 can be reduced even in a case where the thickness of the entire cover member 10 is large.

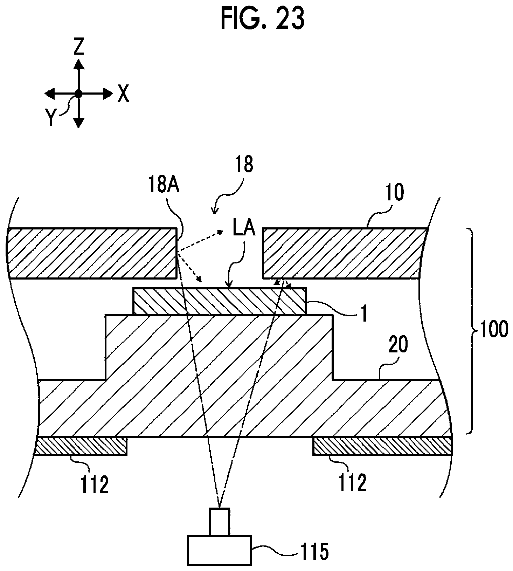

In addition, in the assay apparatus in which the cartridge 100 of the present disclosure is loaded, the illumination unit 115 is disposed above the cartridge 100, but the embodiment of the present disclosure is not limited to this. For example, as shown in Fig. 23, the illumination unit 115, which is an example of the light source, may be provided below the cartridge 100.

In such a case where the observation region LA is illuminated from the illumination unit 115 below the cartridge 100, the observation region LA is irradiated with the transmitted light transmitted through the case body 20 and the assay strip 1. Even in such a case, since the transmitted light is reflected by the inner peripheral wall 18A and the reflected light is diffused, the observation region LA can be illuminated with the scattered light.

In a case where the observation region LA is illuminated from the illumination unit 115 below the cartridge 100, a portion facing the assay strip 1 of the inner surface of the cover member 10 is also preferably a processed surface subjected to rough surface treatment. Accordingly, the scattering effect in a case where the transmitted light is reflected can be enhanced.

In addition, in the cartridge 100 of the present disclosure, the assay region L1, the control region L2, and the color development region L3 are formed in the observation region LA, but the embodiment of the present disclosure is not limited to this. As long as at least the assay region L1 is formed in the observation region LA, the effect of scattering light by the inner peripheral wall 18A can be utilized.

In addition, in the above-described embodiment, the cartridge 100 in which the color development state of the assay region L1 can be visually confirmed has been described as an example, but it is possible to apply the technique of the present disclosure to a cartridge in which the color development state of the assay region cannot be visually confirmed. Examples of such a cartridge include a fluorescence type cartridge in which the assay region emits fluorescence by irradiation with excitation light. In the fluorescent type cartridge, the color development state of the assay region cannot be visually confirmed in a state where the excitation light is not irradiated. However, also in a case where the excitation light is used as the illumination light in the assay region, the light quantity distribution of the excitation light affects the color development state of the fluorescence in the assay region. Therefore, the technique of the present disclosure is also effective for such a cartridge.

The present disclosure is not limited to the above embodiments, and can be implemented with appropriate modifications, such as omitting a configuration or replacing a configuration with a different configuration within the scope that does not deviate from the gist of the present disclosure.

The disclosure of Japanese Patent Application No. 2021-054135 filed on March 26, 2021 is incorporated herein by reference in its entirety.

All literatures, patent applications, and technical standards described in the present specification are incorporated in the present specification by reference to the same extent as in a case where the individual literatures, patent applications, and technical standards are specifically and individually stated to be incorporated by reference.

## Claims

1. An assay cartridge that is attachably and detachably loaded into an immunochromatographic assay apparatus, the assay cartridge comprising:

   a carrier having an assay region in which a color development state changes depending on whether a sample is positive or negative;
   a case in which the carrier is accommodated; and
   an opening portion that is provided in the case, is used for observing an observation region including the assay region, and is illuminated by a light source in the immunochromatographic assay apparatus,
   wherein at least a part of an inner peripheral wall of the opening portion is a processed surface having an angle of 50° or more with respect to a surface of the observation region and having been subjected to a roughening treatment.

2. The assay cartridge according to claim 1, wherein the angle is in a range of 70° or more and 90° or less.

3. The assay cartridge according to claim 1 or 2,

   wherein the opening portion has a rectangular shape, and
   the processed surface is formed on a wall surface of the inner peripheral wall extending in a longitudinal direction.

4. The assay cartridge according to claim 3,

   wherein the assay region is a line-shaped assay line, and
   the assay line is disposed along a direction intersecting the longitudinal direction.

5. The assay cartridge according to any one of claims 1 to 4,
   wherein the processed surface is formed over an entire circumference of the inner peripheral wall.

6. The assay cartridge according to any one of claims 1 to 5,

wherein among parameters defining surface roughness of the processed surface, an arithmetic average height Sa is 100 $\mu$m or less.

7. The assay cartridge according to any one of claims 1 to 6, wherein the roughening treatment is an emboss processing.

8. The assay cartridge according to any one of claims 1 to 7,

   wherein the opening portion has a rectangular shape, and in a case where an opening width in the direction along the surface of the observation region and in a short side direction of the opening portion is denoted as D, and a height of the inner peripheral wall in the direction orthogonal to the opening width is denoted as H,

$$0.5D \le H \le 1.5D.$$

9. The assay cartridge according to any one of claims 1 to 8,

   wherein in a case where the inner peripheral wall of the opening portion is a first inner peripheral wall, the assay cartridge has a second inner peripheral wall that rises in a direction away from the surface along an intersection direction intersecting the surface of the observation region, around the opening portion.

10. The assay cartridge according to claim 9,

    wherein a plane portion extending in a direction along the surface of the observation region is provided around the opening portion, and the second inner peripheral wall rises from an end edge of the plane portion on a side opposite to the opening portion.

11. The assay cartridge according to claim 9,

    wherein in a case where the inner peripheral wall of the opening portion is a first inner peripheral wall, the second inner peripheral wall rises from an end edge of the first inner peripheral wall.

12. The assay cartridge according to any one of claims 9 to 11, wherein at least a part of the second inner peripheral wall is subjected to the roughening treatment.

13. The assay cartridge according to any one of claims 9 to 12, wherein the second inner peripheral wall is inclined at an angle smaller than the angle of the first inner peripheral wall.

14. The assay cartridge according to any one of claims 1 to 13,

    wherein a structure having a convex shape in a direction away from the surface of the observation region is provided around the opening portion, and at least a part of an outer peripheral wall of the structure is subjected to the roughening treatment.

# FIG. 1

# FIG. 2

FIG. 3A

FIG. 3B

EP 4 317 973 A1

# FIG. 4

DOWNSTREAM ◄—————————————————————————— UPSTREAM

# FIG. 5

| S1 | SAMPLE SPOTTING |
| S2 | SUPPLY OF FIRST REAGENT |
| S3 | Wait (DEVELOPMENT OF SAMPLE, LABELING SUBSTANCE, AND FIRST REAGENT) |
| S4 | Wait (DEVELOPMENT OF FIRST REAGENT) |
| S5 | SUPPLY OF SECOND REAGENT |
| S6 | COMPLETION OF SIGNAL AMPLIFICATION |

FIG. 6

FIG. 7

# FIG. 8

# FIG. 9

## FIG. 10

## FIG. 11

## FIG. 12

# FIG. 13

# FIG. 14

## COMPARATIVE EXAMPLE

# FIG. 15

# FIG. 16

# FIG. 17

Z
X
Y

K1

10

18A

N

(ABOUT 90°=) θ 1

K2

LA

1

# FIG. 18

Z
X
Y

18C    18    18C    10
18A    LA    18A    1

# FIG. 19

Z
X
Y

18C    K1
K10
(θ1>)θ2    18A    N
θ1    LA    1
K2

FIG. 20

# FIG. 21

# FIG. 22

# FIG. 23

## INTERNATIONAL SEARCH REPORT

| | |
|---|---|
| International application No. | |
| | **PCT/JP2022/010715** |

**A. CLASSIFICATION OF SUBJECT MATTER**

*G01N 33/543*(2006.01)i
FI:  G01N33/543 521

According to International Patent Classification (IPC) or to both national classification and IPC

**B. FIELDS SEARCHED**

Minimum documentation searched (classification system followed by classification symbols)

G01N33/543

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 2002-22742 A (HAMAMATSU PHOTONICS KK) 23 January 2002 (2002-01-23) entire text, all drawings | 1-14 |
| A | JP 2013-238543 A (KONICA MINOLTA, INC.) 28 November 2013 (2013-11-28) entire text, all drawings | 1-14 |
| A | JP 2015-31610 A (DENKA SEIKEN CO., LTD.) 16 February 2015 (2015-02-16) entire text, all drawings | 1-14 |
| A | JP 2011-89917 A (AISIN SEIKI CO., LTD.) 06 May 2011 (2011-05-06) entire text, all drawings | 1-14 |
| A | JP 2002-22743 A (HAMAMATSU PHOTONICS KK) 23 January 2002 (2002-01-23) entire text, all drawings | 1-14 |
| A | JP 2010-14620 A (TOYO KOHAN CO., LTD.) 21 January 2010 (2010-01-21) entire text, all drawings | 1-14 |
| A | JP 9-68532 A (TOPPAN PRINTING CO., LTD.) 11 March 1997 (1997-03-11) entire text, all drawings | 1-14 |

☐ Further documents are listed in the continuation of Box C.     ☑ See patent family annex.

| | |
|---|---|
| \* Special categories of cited documents: | "T" later document published after the international filing date or priority date and not in conflict with the application but cited to understand the principle or theory underlying the invention |
| "A" document defining the general state of the art which is not considered to be of particular relevance | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | "&" document member of the same patent family |
| "P" document published prior to the international filing date but later than the priority date claimed | |

| Date of the actual completion of the international search | Date of mailing of the international search report |
|---|---|
| **15 April 2022** | **10 May 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**
**Information on patent family members**

International application No.

**PCT/JP2022/010715**

| Patent document cited in search report | | | Publication date (day/month/year) | Patent family member(s) | | | Publication date (day/month/year) |
|---|---|---|---|---|---|---|---|
| JP | 2002-22742 | A | 23 January 2002 | (Family: none) | | | |
| JP | 2013-238543 | A | 28 November 2013 | (Family: none) | | | |
| JP | 2015-31610 | A | 16 February 2015 | US | 2016/0187329 | A1 | |
| | | | | entire text, all drawings | | | |
| | | | | WO | 2015/016310 | A1 | |
| | | | | CN | 105408749 | A | |
| | | | | KR 10-2016-0036574 | | A | |
| JP | 2011-89917 | A | 06 May 2011 | (Family: none) | | | |
| JP | 2002-22743 | A | 23 January 2002 | (Family: none) | | | |
| JP | 2010-14620 | A | 21 January 2010 | (Family: none) | | | |
| JP | 9-68532 | A | 11 March 1997 | (Family: none) | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2013238543 A
- US 6020117 A
- JP 2021054135 A